# FASCICULE DE BREVET EUROPEEN

(11) **EP 1 523 243 B1**
(45) Date de publication et mention de la délivrance du brevet: **10.01.2007**
(21) Numéro de dépôt: 03762713.0
(22) Date de dépôt: 30.06.2003
(51) Int. Cl.: A23J 1/20, A23C 9/146, A23L 1/305, A61K 38/40, A61K 38/17

(54) **ISOLAT DE PROTEINES DE LAIT ET PROCEDE POUR SA PREPARATION**
MILCHEIWEISSISOLAT UND VERFAHREN ZUR HERSTELLUNG
MILK PROTEIN ISOLATE AND METHOD FOR PREPARING SAME

(30) Priorité: 02.07.2002 FR 0208234
(43) Date de publication de la demande: 20.04.2005
(73) Titulaire: COMPAGNIE LAITIERE EUROPEENNE, F-50890 Condé-sur-Vire (FR)
(72) Inventeur: SOUPPE, Jérôme, F-35700 RENNES (FR)
(74) Mandataire: Corizzi, Valérie
(86) Numéro de dépôt international: PCT/FR2003/002015
(87) Numéro de publication internationale: WO 2004/004482

(56) Documents cités:
- EP-A- 0 704 218
- EP-A- 1 010 430
- WO-A-93/13676
- WO-A-97/27757
- WO-A-99/15024
- FR-A- 2 443 867
- US-A- 5 149 647
- US-A- 6 096 870
- PATENT ABSTRACTS OF JAPAN vol. 1996, no. 10, 31 octobre 1996 (1996-10-31) & JP 08 165249 A (SNOW BRAND MILK PROD CO LTD), 25 juin 1996 (1996-06-25) cité dans la demande
- PATENT ABSTRACTS OF JAPAN vol. 1997, no. 11, 28 novembre 1997 (1997-11-28) & JP 09 187250 A (SNOW BRAND MILK PROD CO LTD), 22 juillet 1997 (1997-07-22) cité dans la demande
- HAHN R ET AL: "Bovine whey fractionation based on cation-exchange chromatography" JOURNAL OF CHROMATOGRAPHY A, ELSEVIER SCIENCE, NL, vol. 795, no. 2, 6 février 1998 (1998-02-06), pages 277-287, XP004108665 ISSN: 0021-9673
- CHIU C K ET AL: "Fractionation of lactoperoxidase and lactoferrin from bovine whey using a cation exchange membrane." JOURNAL OF FOOD SCIENCE 1997 DEP. OF FOOD SCI., UNIV. OF WISCONSIN, MADISON, WI 53706-1519, USA, vol. 62, no. 5, pages 996-1000, XP002233814

## Description

La présente invention a pour objet un nouveau procédé d'isolement de protéines de lait, la composition, isolat de protéines de lait ou fraction protéique laitière, en résultant et ses applications, notamment alimentaires et pharmaceutiques.

On connaît plusieurs documents de l'art antérieur décrivant la purification de protéines de lait.

Le document EP-253395 décrit l'obtention d'une lactoferrine bovine de haute pureté (>80% en une étape ou >98% en deux étapes) par adsorption de lait ou de lactosérum sur une résine échangeuse de cations comprenant des radicaux carboxyméthyle, puis rinçage et désorption de la lactoferrine.

Le document EP-348508 décrit également un procédé de préparation d'une lactoferrine bovine de haute pureté (>95%) par adsorption de lait ou de lactosérum sur une résine échangeuse de cations de type polysaccharide comprenant des fonctions ester d'acide sulfurique et élution par une solution aqueuse de sel.

Le document EP-298875 décrit un procédé permettant d'isoler certaines protéines du lactosérum par adsorption sur un support minéral poreux sous forme de grains, ces grains étant recouverts d'une couche de polysaccharide aminé présentant en surface des groupements fonctionnels acides, tels que des groupements carboxyliques ou sulfoniques.

Le document EP-418704 décrit un procédé pour purifier séquentiellement la lactoferrine et la lactoperoxydase par élution sur une colonne chargée avec une résine polysaccharide greffée par des groupes acide sulfonique.

Le document US-6,096,870 décrit un procédé de séparation séquentielle de protéines du petit lait par élution sur des résines cationiques.

Le document EP-1017286 décrit un procédé de séparation séquentielle de protéines du petit-lait par chromatographie à flux radial.

La lactoferrine et la lactoperoxydase sont des protéines du lait dotées de propriétés intéressantes : leur capacité à lier le fer leur confère un rôle d'agent anti-bactérien contre les bactéries dont le métabolisme requiert d'importantes quantités de fer. La lactoperoxydase est indispensable au marquage protéique par l'iode. La lactoferrine favorise la croissance des lymphocytes et favorise l'absorption du fer par l'organisme, elle régule la différentiation des leucocytes et elle inhibe la peroxydation des lipides.

Toutefois, tous ces documents concernent des procédés visant à obtenir une lactoferrine et/ou une lactoperoxydase les plus pures possibles, c'est-à-dire des procédés visant à réduire le plus possible la proportion des autres protéines initialement présentes dans le lait ou le lactosérum. En outre, les conditions de fixation de la matière première et d'élution des protéines sont des conditions douces, avec des flux de matières lents. En revanche, l'objectif que s'est fixé la demanderesse est l'obtention d'une fraction protéique laitière comprenant, entre autres constituants, de la lactoferrine et de la lactoperoxydase, mais comprenant surtout une proportion des autres protéines plus élevée que dans le lait ou le lactosérum de départ. Pour cela la demanderesse a mis au point un procédé avec des flux beaucoup plus élevés que ceux décrits dans les documents analysés ci-dessus, ce procédé permettant une fixation plus sélective vis-à-vis de certaines protéines.

Le document WO93/13676 divulgue un procédé permettant d'isoler la lactoferrine et la lactoperoxydase par passage sur une résine échangeuse de cations à flux élevé, à partir de petit-lait. Ce procédé se distingue du procédé selon la présente invention par le fait que le flux est plus élevé que dans le procédé selon l'invention (supérieur à 5 m/h) et qu'il vise à purifier la lactoferrine et la lactoperoxydase, et non pas à obtenir une fraction protéique laitière particulière contenant un taux relativement élevé des autres protéines et dotée de propriétés biologiques améliorées.

On connaît également, par le document EP-704218 un agent destiné à renforcer les os, cet agent comprenant une fraction protéique basique ou une fraction peptidique basique, issues du lait et obtenues par passage du lait sur une résine cationique et élution. Toutefois, les conditions d'adsorption et d'élution décrites dans ce document sont distinctes de celles employées dans le procédé selon l'invention et permettent d'isoler une fraction protéique distincte de celle obtenue selon la présente invention.

Les documents JP-8165249, JP-9187250, JP-9294537 et EP-1010430 décrivent des compostions destinées au renforcement des os et/ou de prévention des maladies parodontales, ces compositions comprenant une fraction protéique basique dérivée du lait obtenue par adsorption du lait et élution sur une résine cationique. Toutefois, les conditions d'adsorption et d'élution décrites dans ces documents sont distinctes de celles employées dans le procédé selon l'invention et permettent d'isoler une fraction protéique distincte de celle obtenue selon la présente invention.

Ainsi, c'est avec étonnement que la demanderesse a découvert un nouveau procédé d'isolement de protéines de lait permettant d'obtenir une fraction protéique laitière dotée de propriétés biologiques améliorées, notamment une fraction protéique laitière favorisant la croissance des ostéoblastes et l'inhibition de la prolifération des pré-ostéoclastes et des ostéoclastes.

L'invention a pour objet un procédé d'isolement de protéines de lait à partir de lait ou d'un lactosérum comportant les étapes suivantes :
a) le lait ou le lactosérum est stérilisé et dégraissé ;
b) la fraction laitière issue de l'étape a) est passée sur une résine échangeuse de cations conditionnée dans une colonne d'élution ;
c) la fraction retenue sur la résine est éluée par une solution aqueuse salée ;
d) l'éluat résultant de l'étape c) est dessalé, de préférence par ultrafiltration, et diafiltration puis stérilisé, de préférence par microfiltration.

Ce procédé étant caractérisé en ce que :
α) la résine échangeuse de cations est une résine greffée par des fonctions acides fortes ;
   le paramètre BV désignant le rapport du volume de matière première au volume de résine humide dans la colonne,
   le paramètre SV désignant le rapport du débit d'alimentation de la colonne au volume de résine humide dans la colonne,
   le paramètre LV désignant le rapport du débit d'alimentation de la colonne à la section de la colonne,
β) au cours de l'étape b), les paramètres de fixation ont les valeurs suivantes :
   - BV_{f} est compris entre 50 et 400, de préférence entre 80 et 300;
   - SV_{f} est compris entre 2 et 40 h⁻¹;
   - LV_{f} est supérieur ou égal à 1 m/h et inférieur ou égal à 5 m/h.
γ) au cours de l'étape c), les paramètres d'élution ont les valeurs suivantes :
   - BVₑ est compris entre 1,5 et 7 ;
   - LVₑ est inférieur à 1m/h, de préférence inférieur à 0,5 m/h.

On peut utiliser comme produit de départ dans le procédé selon l'invention soit du lait, soit du lactosérum, préférentiellement issus de la vache. Le lactosérum est le liquide résiduel obtenu après l'extraction des protéines et de la matière grasse du lait ou du petit-lait. On distingue en général trois catégories de lactosérum. Les deux premières catégories sont classées selon l'acidité du lactosérum qui peut être inférieure ou supérieure à 1,8 g d'acide lactique/l : le lactosérum doux, issu de la fabrication de fromage à pâte pressée cuite ou non cuite (emmenthal, saint-paulin etc.) et le lactosérum acide, issu de la caséine ou d'autres fromages obtenus par coagulation mixte ou lactique (pâtes molles, pâtes fraîches). La composition moyenne du lactosérum doux est à titre indicatif, pour 61 g de matières sèches par kg de lactosérum, de 42 à 48 g de lactose, 8 g de protéines, 2 g de graisses, 5 à 7 g de minéraux, 1 à 5 g d'acide lactique et le reste en minéraux et vitamines.

On connaît également le lactosérum idéal obtenu par microfiltration du lait sur un support de porosité moyenne de 0,1 µm.

Selon une première variante de l'Invention, on utilise comme produit de départ du lait, et avantageusement du lait de vache, dont la composition permet, par le procédé selon l'invention, l'obtention d'un isolat de protéines ayant des propriétés biologiques plus intéressantes. Cette variante permet également d'obtenir un rendement en protéines supérieur aux variantes utilisant le lactosérum comme produit de départ.

Selon une seconde variante préférée de l'Invention, on utilise comme produit de départ du lactosérum acide de caséinerie. Cette variante représente un avantage économique important dans la mesure où le produit de départ est un sous-produit issu de l'exploitation industrielle et donc d'un faible coût.

Dans la première étape du procédé, le lait ou le lactosérum sont stérilisés et dégraissés par écrémage, de façon connue :
L'écrémage du lait désigne la séparation de la crème du lait, quel que soit le procédé mis en oeuvre pour obtenir cette séparation.

De façon traditionnelle, la fabrication de la crème se fait selon un processus naturel : lorsque le lait repose, les éléments qui le composent se séparent en fonction de leur densité. Les globules de matière grasse étant plus légers que l'eau remontent à la surface pour former une couche de crème. En production industrielle, la formation de la crème est accélérée par passage du lait dans une écrémeuse centrifuge.

De façon habituelle, la pasteurisation est faite par un chauffage contrôlé de courte durée de façon à éliminer les germes pathogènes éventuellement présents dans la crème. Avantageusement, la pasteurisation est faite à une température comprise entre 65 et 95°C, préférentiellement entre 80 et 95°C. On peut par exemple traiter le lait ou le lactosérum pendant 15 secondes à une température comprise entre 65 et 82°C. On peut également stériliser le lait ou le lactosérum de départ par microfiltration sur un filtre doté de pores d'un diamètre allant de 0,1 à 2 µm.

La matière première stérile et dégraissée est alors passée sur une résine échangeuse de cations. Selon l'invention la résine échangeuse de cations est une résine greffée par des fonctions acides fortes et ayant une capacité d'échange d'ions comprise entre 200 et 1000 µE/ml, de préférence entre 400 et 700 µE/ml. Par fonction acide forte on entend une fonction acide dotée d'un pKa ≤ 2. Notamment elle peut être greffée par des fonctions acide sulfonique, généralement sous forme de sels de sulfonate pour leur mise en oeuvre, la nature du sel étant déterminée par la solution qui a servi à conditionner la colonne avant la mise en oeuvre du procédé. Préférentiellement, on choisit un greffage par des groupements aromatiques ou aliphatiques porteurs de fonctions acide sulfonique, encore plus préférentiellement sous forme de sels de propyl sulfonate. La résine sur laquelle sont greffées les fonctions acides sulfoniques peut être de toute nature, notamment polyacrylique ou polystyrène. La granulométrie de la résine est avantageusement comprise entre 100 µm et 900 µm, de préférence entre 200 et 750 µm, encore plus préférentiellement entre 250 et 600 µm. La résine utilisable selon l'invention doit préférentiellement présenter une densité supérieure à 1,15.

Parmi les résines commercialement disponibles utilisables dans la présente invention, on peut citer notamment : la résine Trisacryl SP ® commercialisée par la société BIOSEPRA, la résine MacroPrep High S ® commercialisée par la société BioRad. De préférence, on choisit une résine polystyrène greffée par des fonctions alkyl ou aryl sulfonate.

La résine est conditionnée dans une colonne avant son utilisation, de façon connue de l'homme du métier, par traitement par une solution désinfectante et rinçage afin d'éviter la contamination par des micro-organismes. Elle est ensuite éventuellement équilibrée par passage d'une solution tampon et rinçage.

L'étape b) de fixation de la matière première, lait ou lactosérum, stérile et dégraissée, se déroule dans les conditions préférentielles suivantes :
La résine est conditionnée dans une colonne dont la température est maintenue entre 2 et 15°C, préférentiellement entre 4 et 12°C. Préférentiellement, la colonne est alimentée en matière première par le bas. Avantageusement on travaille en lit fluidisé. De préférence les paramètres de fixation sont ajustés de façon à ce que l'une ou plusieurs des conditions suivantes soient vérifiées :
   - BV_{f} est compris entre 80 et 150, préférentiellement entre 80 et 120;
   - SV_{f} est compris entre 5 et 40 h⁻¹, préférentiellement entre 8 et 20 h⁻¹, encore plus préférentiellement entre 8 et 15 h⁻¹;
   - LV_{f} est compris entre 3 et 4,8 m/h, préférentiellement entre 3,2 et 4 m/h.

De façon préférentielle, l'ensemble des conditions suivantes est vérifié au cours de l'étape b) :
- BV_{f} est compris entre 80 et 120;
- SV_{f} est compris entre 8 et 15 h⁻¹;
- LV_{f} est compris entre 3 et 4,8 m/h.
au cours de l'étape c) :
- BVₑ est compris entre 3 et 7
- LVₑ est inférieur à 1 m/h.

Par l'échange d'ions, les protéines de la fraction laitière utilisée comme produit de départ viennent se fixer sur les fonctions acides de la résine. Le choix des paramètres de fixation selon l'invention permet d'opérer une fixation sélective des protéines sur la colonne. Dans des conditions classiques de fixation du lait sur une résine cationique, la lactoferrine et la lactoperoxydase, majoritaires, se fixent préférentiellement sur la résine. Dans le procédé selon l'invention, les autres protéines, minoritaires, sont favorisées par les conditions de fixation définies ci-dessus et leur proportion dans le mélange de protéines fixées sur la résine est significativement supérieure à leur proportion dans le produit de départ. Le procédé selon l'invention permet ainsi d'isoler une fraction laitière ayant une composition en protéines nouvelle par rapport aux compositions protéiques laitières de l'art antérieur et présentant des propriétés biologiques intéressantes. L'étape c) d'élution des protéines fixées, se déroule dans les conditions préférentielles suivantes :
La résine est maintenue à une température comprise entre 2 et 15°C, préférentiellement entre 4 et 12°C. Préférentiellement, la colonne est alimentée en matière première (solution aqueuse salée) par le haut. La solution aqueuse saline employée pour la mise en oeuvre de l'invention est généralement une solution d'un chlorure d'un métal alcalin tel que K+, Na+, Ca+, Mg+. De préférence on emploie une solution aqueuse de chlorure de sodium. Avantageusement, la solution aqueuse de sel a une concentration comprise entre 2 et 25%, préférentiellement de 5 à 15% en poids de sel par poids de liquide. De préférence, la force ionique de la solution aqueuse de sel est comprise entre 1 et 2 M. Le pH de la solution d'élution est généralement compris entre 6 et 7, avantageusement entre 6,5 et 7.

De préférence les paramètres d'élution vérifient l'une ou plusieurs des conditions suivantes :
- BVₑ est compris entre 3 et 7 et préférentiellement entre 3 et 5 ;
- LVₑ est inférieur à 0,5 m/h.

De façon connue, la colonne de résine est lavée avant une nouvelle utilisation.

Après cette étape, l'éluat obtenu contenant le mélange de protéines de lait est soumis de façon connue à une ou plusieurs étapes d'ultrafiltration et de diafiltration destinées à éliminer les sels. D'autres moyens connus de l'homme du métier tels que l'électrodialyse ou le passage sur des résines anioniques et cationiques faibles peuvent être employés dans cette étape en remplacement de l'ultrafiltration et de la diafiltration. De préférence, ce traitement est effectué jusqu'à l'obtention d'un perméat ayant une conductivité inférieure à 15 mS. Tout autre procédé permettant d'éliminer les sels, comme notamment l'électrodialyse, peut être utilisé en remplacement de cette étape. La solution est alors soumise à une microfiltration destinée à stériliser le rétentat d'ultrafiltration avant séchage. D'autres moyens techniques peuvent être employés pour stériliser la fraction laitière obtenue à cette étape, notamment un traitement thermique adapté, des ultrasons ou des champs électriques pulsés.

Préférentiellement, le produit dessalé et stérilisé est séché de façon à obtenir la fraction laitière issue du procédé de l'invention sous forme d'une poudre, ce qui permet ensuite son conditionnement et son stockage. De façon connue, le séchage peut être fait par lyophilisation ou par atomisation.

La fraction protéique laitière, préférentiellement issue du lait de vache et obtenue par le procédé selon l'invention est nouvelle. Sous forme sèche, elle est caractérisée en ce qu'elle est dotée :
- d'une teneur en protéines supérieure à 90%,
- d'une teneur en sels minéraux inférieure à 1%,
- d'une teneur en matières grasses inférieure à 1%,
- d'une teneur en lactose inférieure à 1%,
- d'une teneur en humidité inférieure à 5%,
- d'une teneur en lactoferrine inférieure à 80%,
- d'un pH en solution à 2% compris entre 6 et 7,5,
- d'une pureté spectrophotométrique en UV-visible définie par un ratio DO⁴¹²/DO²⁸⁰<0,15.
- d'au moins 1% de protéines ayant un point isoélectrique supérieur ou égal à 8, les pourcentages étant donnés en poids par rapport au poids de matière sèche de la fraction laitière selon l'invention.

Dans le cas où le produit de départ utilisé est du lait et non un lactosérum, le produit obtenu par le procédé de l'invention se caractérise par les conditions supplémentaires :
- présence d'au moins 40% de protéines ayant un point isoélectrique supérieur ou égal à 8,
- la teneur en lactoferrine est supérieure à 30% et inférieur à 80%,
- l'activité lactoperoxydase est supérieure ou égale à 120 unités ABTS par mg d'isolat (ABTS=2,2'-Azino-bis-(3-éthyl Benzo Thiazoline 6-Sulfonic Acid).

La mesure de DO à 412 nm donne une évaluation quantitative de la lactoferrine et de la lactoperoxydase présentes dans la fraction protéique laitière.

La mesure de DO à 280 nm donne une évaluation quantitative de la totalité des protéines présentes dans la fraction protéique laitière.

Le ratio DO⁴¹²/DO²⁸⁰<0,15 montre que la lactoferrine et la lactoperoxydase sont sous-représentées par rapport aux autres protéines dans la composition selon l'invention par comparaison avec les proportions dans lesquelles elles sont présentes dans le lait et dans les fractions protéiques laitières de l'art antérieur.

De préférence, la fraction protéique laitière obtenue par le procédé selon l'invention répond au moins à l'une des caractéristiques suivantes :
- une teneur en protéine supérieure à 95%,
- une teneur en sels minéraux inférieure à 0,5%,
- une teneur en matières grasses inférieure à 0,5%,
- une teneur en lactose inférieure à 0,5%,
- une teneur en humidité inférieure à 4%,
- un pH en solution à 2% compris entre 6 et 7,2,
- une pureté spectrophotométrique en UV-visible définie par un ratio DO⁴¹²/DO²⁸⁰<0,1.
- contient au moins 1% de protéines ayant un point isoélectrique compris entre 8,2 et 8,7.

Dans le cas où le produit de départ utilisé est du lait et non du lactosérum, le produit obtenu par le procédé de l'invention se caractérise par la condition préférentielle additionnelle :
- la teneur en lactoferrine est supérieure à 50% et inférieure à 80%.

Les fractions protéiques laitières selon l'invention présentent des propriétés avantageuses : notamment elles favorisent la croissance des cellules ostéoblastiques et celle des cellules intestinales.

Les fractions protéiques laitières de l'invention sont également efficaces pour inhiber la croissance des ostéoclastes et des pré-ostéoclastes.

Ces propriétés ainsi que les propriétés déjà connues des fractions protéiques laitières de l'art antérieur permettent d'envisager l'utilisation de ces compositions dans de nombreuses applications, notamment dans les domaines alimentaire et pharmaceutique. L'invention a donc également pour objet toute composition alimentaire, pharmaceutique et tout produit d'hygiène comprenant une fraction protéique laitière selon l'invention.

Une composition alimentaire selon l'invention pourra par exemple être un lait alimentaire, en particulier un lait destiné à l'alimentation infantile, obtenu par simple réhydratation de la poudre de fraction protéique laitière selon l'invention. L'invention a donc pour objet l'utilisation d'une fraction protéique laitière selon l'invention pour la préparation d'un lait alimentaire.

L'invention a également pour objet des compositions alimentaires comprenant une fraction protéique laitière selon l'invention et d'autres ingrédients alimentaires. On peut en particulier envisager d'additionner la fraction protéique laitière selon l'invention à un lait de vache afin d'enrichir celui-ci en certaines protéines. La présence de calcium dans les aliments comprenant la fraction protéique laitière selon l'invention sera particulièrement bénéfique dans la mesure ou cette fraction protéique laitière améliore l'absorption du calcium par l'organisme humain, notamment la fixation du calcium dans le tissu osseux.

L'invention a donc également pour objet l'association d'une fraction protéique laitière selon l'invention avec du calcium.

L'invention porte également sur les kits alimentaires comprenant plusieurs constituants conditionnés de façon isolée, destinés à une préparation extemporanée de l'aliment et comprenant de la poudre de fraction protéique laitière selon l'invention.

De tels aliments peuvent être utilisés pour la prévention de pathologies telles que : retard de croissance, ostéoporose, fragilité osseuse, fractures osseuses, rhumatismes, arthrose, maladies périodontales, déficience de la barrière intestinale ... ,

Une composition pharmaceutique selon l'invention, comprenant au moins une fraction protéique laitière selon l'invention et un support pharmaceutiquement acceptable peut être utilisée dans le traitement d'une ou plusieurs des pathologies suivantes : retard de croissance, ostéoporose, fragilité osseuse, fractures osseuses, rhumatismes, arthrose, maladies périodontales, déficience de la barrière intestinale ...

Bien entendu une telle composition peut en outre comporter un ou plusieurs autres actifs thérapeutiques. Du calcium peut avantageusement être associé à la fraction protéique laitière selon l'invention. Une telle association fait partie de la présente invention. En effet, une fraction protéique laitière selon l'invention permet d'améliorer l'absorption du calcium dans l'organisme, notamment la fixation du calcium dans le tissu osseux, ce qui permet d'améliorer l'effet renforçateur des os.

Du calcium avec la vitamine D peut aussi avantageusement être associé à la fraction protéique laitière selon l'invention. Une telle association fait aussi partie de la présente invention. En effet, la vitamine D améliore l'absorption intestinale du calcium et une fraction protéique laitière selon l'invention permet d'améliorer la fixation du calcium dans le tissu osseux. Cet effet synergie permet particulièrement d'améliorer la santé des os.

Des compositions pharmaceutiques ou des compléments alimentaires selon l'invention peuvent être administrés sous toute forme appropriée telle que sous forme de poudre, de granulés, de comprimés, de gélules, de boisson, comme par exemple en solution ou en sirop. La fréquence d'administration et la dose à administrer sont adaptés de façon connue en fonction du poids et de l'âge de l'individu.

Sont également inclues dans la présente invention, les produits d'hygiène, notamment les produits destinés à l'hygiène de la cavité buccale, tels que les dentifrices sous forme de gel ou de pâte, les bains de bouche, les gommes à mâcher, comprenant une fraction protéique laitière selon l'invention.

### EXEMPLES :

### I- Préparation des isolats de protéines de lait

### Conditions générales :

La résine employée dans les exemples ci-dessous est une résine Trisacryl SP ®. Elle subit les traitements de conditionnement suivants avant sa mise en oeuvre initiale : mise en contact avec une solution aqueuse à 0,4% du désinfectant ASEPTO ®, à raison de 3 litres de désinfectant par kilo de résine humide, suivie d'un rinçage. Mise en équilibre par passage d'un tampon acétate (80 mM, pH=5,3) contenant du chlorure de calcium (33 g/l) et du chlorure de potassium (50 g/l), à raison de 4 litres de ce tampon par kilo de résine humide. Enfin la résine est rincée à l'eau jusqu'à l'obtention d'un éluat ayant un pH compris entre 6 et 7 et une conductivité inférieure à 5 mS.

Après chaque séquence de fixation de matière première et d'élution, la résine est nettoyée par traitement enzymatique à la protéase alcaline de Bacillus licheniformis en tampon à pH=8, à 60°C pendant 2 heures, puis traitement par une solution de NaCl à 100g par litre pendant trente minutes (répété 2 fois). Avant sa réutilisation, elle est soumise au traitement de conditionnement exposé ci-dessus.

L'ultrafiltration est effectuée sur l'éluat à une température comprise entre 5 et 10°C sur des membranes organiques dont le seuil de coupure est de 10kD. Le facteur de concentration volumique pratiqué est compris entre 10 et 60, de façon à obtenir un rétentat ayant l'extrait sec le plus élevé possible, de préférence entre 15 et 20%. La diafiltration est effectuée à une température comprise entre 15 et 25°C sur les mêmes membranes, en utilisant un volume d'eau déminéralisée compris entre 8 et 10 fois le volume de rétentat obtenu à l'issue de l'ultrafiltration jusqu'à atteindre une conductivité du perméat inférieure à 15mS.

La microfiltration est effectuée à 35°C à partir du rétentat issu de l'ultrafiltration /diafiltration sur des membranes céramiques de seuil de coupure de 1,4 µm.

Le séchage du perméat de microfiltration est effectué sur une tour d'atomisation à turbines avec une température d'entrée de 140°C et une température de sortie de 80°C.

### Caractéristique de la résine :

La résine SPEC 70 est constituée d'un support chimiquement inerte et mécaniquement résistant.

Chimiquement, le support est fabriqué par polymérisation d'AMPS : 2-acrylamide 2-méthyl propane sulfonic acid. La résine a une capacité d'échange d'ions entre 400 et 700 µE/ml et une capacité de fixation protéique mesurée sur la lactoferrine (minimum 20 mg/ml) ou la lactoperoxydase (minimum 40 mg/ml) ou le lysozyme (minimum 70 mg/ml) ; sa densité est supérieure à 1,15 et sa granulométrie se situe entre 250 et 560 µm.

### Exemple 1 :

300000 litres de lait écrémé et traité thermiquement à 68°C pendant 15 secondes, sont passés à 10°C sur 3000 litres de résine échangeuse de cations (Référence SPEC 70 fournie par la Société BIOSEPRA) à un débit de 14000 litres /h en flux ascendant dans deux colonnes fonctionnant en parallèle et ayant chacune un rayon de 1 mètre.

Les protéines fixées sur le résine sont éluées par 9000 litres d'une solution de chlorure de sodium à 10% en flux descendant.

L'éluat obtenu présente un pH de 6,5 et une extrait sec de 80 g/kg. Il est passé sur membranes d'ultrafiltration de seuil de coupure de 10 kD et de 17 m² de surface fournies par DSS, à 11°C avec un flux de perméation de 161/h/m² jusqu'à obtenir un facteur de concentration volumique de 7.

Le rétentat a un pH de 5,9 et un extrait sec de 170 g/kg ; afin d'éliminer le sel, ce rétentat est ensuite diafiltré sur les mêmes membranes à 25°C avec de l'eau à hauteur de 70% du volume d'éluat mis en oeuvre et jusqu'à obtenir un facteur de concentration volumique de 9,5 en final avec un flux de perméation de 161/h/m².

Le rétentat diafiltré a un pH de 6,5 et un extrait sec de 95 g/kg ; il est alors passé sur membranes de microfiltration de porosité de 1,4 µm à 30°C avec un flux de perméation de 320 l/h/m². Le perméat de cette microfiltration présente un pH de 6,6 et un extrait sec de 75 g/kg.

Ce perméat est ensuite séché sur une tour d'atomisation munie d'une turbine; la température d'entrée en tour est de 140°C et la température de sortie est de 80°C.

L'isolat poudre ainsi obtenu présente les caractéristiques suivantes:
- Humidité : 4,7%,
- Protéines (Nx6,38) : 96,2% dont :
   Lactoferrine : 54%
   Lactoperoxydase : 125 unité ABTS/mg
      - Cendres : 0, 1 % dont :
      Na : 0,02%
      Cl : 0,44%
- Pureté spectrophotométrique : DO⁴¹²/DO²⁸⁰ = 0,06,

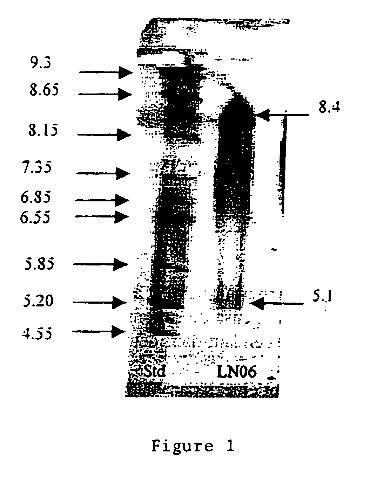

Le profil électrophorétique obtenu par isofocalisation avec révélation au nitrate d'argent est illustré par la figure 1. Sur la figure 1 on compare la répartition des points isoélectriques de la fraction protéique laitière selon l'invention (notée LN06) et un produit de référence noté Std qui est un kit de calibration protéique commercialisé par la Société Pharmacia sous la référence 17047101 et dénommé « Isoelectric Focusing Calibration Kit Broad PI 3-10 ».On constate que sont majoritairement présentes des protéines ayant des points isoélectriques élevés (autour de 8,4). Ce produit comporte environ 50% en poids de lactoferrine par rapport au poids total de l'isolat.

### Exemple 2 :

4,4 litres de sérum acide natif de caséinerie (pH 4,7, extrait sec 65 g/kg) sont passés à 10°C sur 22 ml de résine échangeuse de cations (Référence SPEC 70 fournie par la Société BIOSEPRA) à un débit de 400 ml/h en flux descendant dans une colonne ayant un diamètre de 15 mm.

Les protéines fixées sur la résine sont éluées par 90 ml d'une solution de chlorure de sodium à 10% en flux descendant.

Afin d'éliminer le sel, l'éluat obtenu (100 ml) est ensuite dialysé contre de l'eau déminéralisée pendant 48 heures à 4°C.

Le produit dialysé présente les caractéristiques suivantes:
- Extrait sec : 1,5 g/kg,
- pH : 5,4,
- Conductivité : 24 µS,
- DO⁴¹²<0,005,
- DO²⁸⁰ = 4,6 (0,46 dilué 10 fois).

Le profil électrophorétique permet de constater qu'au moins 1% des protéines présentes ont un point isoélectrique supérieur ou égal à 8. Ce produit contient environ 3% de lactoferrine en poids par rapport au poids total de l'isolat de protéines.

### II- Tests biologiques

### A- Méthodes générales

### 1. Tests de prolifération des cellules MC3T3 (lignée cellulaire ostéoblastique)

Les cellules MC3T3 sont cultivées à 37 °C en atmosphère humide contenant 5 % de CO₂. Le milieu de culture utilisé est de l'□-MEM (alpha-Modified Eagle Medium) supplémenté à l'aide de 10% de sérum de veau foetal et de 50 U/ml de pénicilline et 50 µg/ml streptomycine.
Pour tester l'effet des différentes protéines sur la croissance cellulaire, les cellules sont ensemencées sur plaques 48 puits à une densité de 5 x 10⁴ cellules/cm². 48 heures après ensemencement, les protéines sont ajoutées aux cellules. Les solutions protéiques sont préparées dans le milieu de culture à une concentration de 10 mg/ml puis filtrées sur filtre 0.22µ et diluées à la concentration désirée dans le milieu de culture juste avant usage. Les cellules sont dénombrées après 72h de culture en évaluant la quantité d'ADN.

### 2. Tests de prolifération des cellules RAW 264.7 (lignée cellulaire pré-ostéoclastique)

Les cellules RAW 264.7 sont cultivées à 37 °C en atmosphère humide contenant 5 % de CO₂. Le milieu de culture utilisé est du DMEM (Dubelcco's Modified Eagle Medium) contenant 25 mM de glucose, 4 mM de glutamine et 1.5 g/litre de bicarbonate supplémenté à l'aide de 10% de sérum de veau foetal et de 50 U/ml de pénicilline et 50 µg/ml streptomycine.
Pour tester l'effet des différentes protéines sur la croissance cellulaire, les cellules sont ensemencées sur plaques 48 puits à une densité de 5 x 10⁴ cellules/cm². 48 heures après ensemencement, les protéines sont ajoutées aux cellules. Les solutions protéiques sont préparées dans le milieu de culture à une concentration de 10 mg/ml puis filtrées sur filtre 0.22µ et diluées à la concentration désirée dans le milieu de culture juste avant usage. Les cellules sont dénombrées après 72 heures de culture en évaluant la quantité d'ADN.

### 3. Test de prolifération des cellules Caco-2 (lignée cellulaire intestinale)

Les cellules Caco-2 sont cultivées à 37 °C en atmosphère humide contenant 5 % de CO2. Le milieu de culture utilisé est du DMEM (Dubelcco's Modified Eagle Medium) contenant 25 mM de glucose supplémenté à l'aide de 15% de sérum de veau foetal, d'1% d'acides aminés non essentiels, de 6 mM glutamine et de 50U/ml de pénicilline et 50µg/ml streptomycine.
Pour tester l'effet des différentes protéines sur la croissance cellulaire, les cellules sont ensemencées sur plaques 48 puits à une densité de 4 x 10⁴ cellules/cm². 48 heures après ensemencement, les protéines sont ajoutées aux cellules. Les solutions protéiques sont préparées dans le milieu de culture à une concentration de 10 mg/ml puis filtrées sur filtre 0.22µ et diluées à la concentration désirée dans le milieu de culture juste avant usage. Les cellules sont dénombrées après 72h de culture en évaluant la quantité d'ADN.

### B- Résultats

Les tests décrits ci-dessus ont été mis en oeuvre sur trois produits :
- un isolat de protéines de lait de vache composé à 90% de lactoferrine (Produit témoin) :T₁,
- un isolat de protéines de lait de vache dont la teneur en lactoferrine est de moins de 0,01% (Produit témoin) :T₂,
- le produit préparé à l'exemple 1 : P₁,
- le produit préparé à l'exemple 2 : P₂.

Les résultats de ces tests sont exposés dans le tableau I:

**TABLEAU I**

| Produit | Concentration | Stimulation de la prolifération des cellules MC3T3 | Inhibition de la prolifération des cellules RAW 264.7 | Stimulation de la prolifération des cellules Caco-2 |
|---|---|---|---|---|
| T1 | 1.0 mg/ml | +65% | -70% | +67% |
| | 0.1 mg/ml | +37% | -30% | +40% |
| T2 | 1.0 mg/ml | +9% | -8% | +9% |
| | 0.1 mg/ml | +6% | -4% | - |
| P1 | 1.0 mg/ml | +36% | -70% | +40% |
| | 0.1 mg/ml | +15% | -22% | +30% |
| P2 | 1.0 mg/ml | +31 % | -32% | +41 % |
| | 0.1 mg/ml | +6% | -17% | +37% |

La concentration désigne la concentration de l'isolat de protéines de lait dans le milieu de culture.

L'état de l'art tend à démontrer que l'activité d'un isolat de protéines de lait est liée au pourcentage de lactoferrine qu'il contient (voir résultats de T₂).

Contrairement à ce préjugé de l'art antérieur, les résultats des tests exposés ci-dessus montrent que des isolats de protéine de lait de l'invention, bien que contenant peu de lactoferrine par rapport au témoin T₁, présentent une activité remarquable dans les tests de croissance cellulaire sur des ostéoblastes et sur des cellules intestinales et d'inhibition de la prolifération des pré-ostéoclastes.

## Revendications

1. Procédé d'isolement de protéines de lait à partir de lait ou d'un lactosérum comportant les étapes suivantes :
a) le lait ou le lactosérum est stérilisé et dégraissé;
b) la fraction laitière issue de l'étape a) est passée sur une résine échangeuse de cations conditionnée dans une colonne d'élution ;
c) la fraction retenue sur la résine est éluée par une solution aqueuse salée;
d) l'éluat résultant de l'étape c) est dessalé et stérilisé.
Ce procédé étant **caractérisé en ce que** :
α) la résine échangeuse de cations est une résine greffée par des fonctions acides fortes ;
le paramètre BV désignant le rapport du volume de matière première au volume de résine humide dans la colonne,
le paramètre SV désignant le rapport du débit d'alimentation de la colonne au volume de résine humide dans la colonne,
le paramètre LV désignant le rapport du débit d'alimentation de la colonne à la section de la colonne,
β) au cours de l'étape b), les paramètres de fixation ont les valeurs suivantes :
- BV_{f} est compris entre 5° et 400;
- SV_{f} est compris entre 2 et 40 h⁻¹ ;
- LV_{f} est supérieur ou égal à 1 m/h et inférieur ou égal à 5 m/h .
γ) au cours de l'étape c), les paramètres d'élution ont les valeurs suivantes :
- BVₑ est compris entre 1,5 et 7;
- LVₑ est inférieur à 1 m/h.

2. Procédé selon la revendication 1, **caractérisé en ce que** le produit de départ est du lait de vache.

3. Procédé selon la revendication 1, **caractérisé en ce que** le produit de départ est un lactosérum acide de caséine.

4. Procédé selon l'une quelconque des revendications précédentes, **caractérisé en ce que** la résine échangeuse de cations est une résine greffée par des fonctions acides de pKa ≤ 2 ayant une capacité d'échange d'ions comprise entre 200 et 1000 µE/ml.

5. Procédé selon la revendication 4, **caractérisé en ce que** la résine est greffée par des fonctions acide sulfonique ou sel de sulfonate.

6. Procédé selon la revendication 5, **caractérisé en ce que** la résine est greffée par des fonctions propyl sulfonique ou propyl sulfonate.

7. Procédé selon l'une quelconque des revendications précédentes, **caractérisé en ce que** la granulométrie de la résine est comprise entre 100 µm et 900 µm, de préférence entre 200 et 750 µm, encore plus préférentiellement entre 250 et 600 µm

8. Procédé selon l'une quelconque des revendications précédentes, **caractérisé en ce que** au cours de l'étape b) de fixation de la matière première, une ou plusieurs des conditions suivantes sont remplies :
- BV_{f} est compris entre 80 et 150 ;
- SV_{f} est compris entre 5 et 40 h⁻¹;
- LV_{f} est compris entre 3 et 4,3 m/h.

9. Procédé selon l'une quelconque des revendications précédentes, **caractérisé en ce que** les conditions suivantes sont vérifiées
au cours de l'étape b) :
- BV_{f} est compris entre 80 et 120;
- SV_{f} est compris entre 8 et 15 h⁻¹;
- LV_{f} est compris entre 3 et 4,8 m/h.
au cours de l'étape c) :
- BVₑ est compris entre 3 et 7
- LVₑ est inférieur à 1 m/h.

10. Procédé selon l'une quelconque des revendications précédentes, **caractérisé en ce que** au cours de l'étape b) la résine est conditionnée dans une colonne dont la température est maintenue entre 2 et 15°C,

11. Procédé selon l'une quelconque des revendications précédentes, **caractérisé en ce que** au cours de l'étape c) d'élution des protéines fixées, l'une au moins des conditions suivantes est remplie :
- BVₑ est compris entre 3 et 5 ;
- LVₑ est inférieur à 0,5 m/h.

12. Procédé selon l'une quelconque des revendications précédentes, **caractérisé en ce que** au cours de l'étape c) la résine est conditionnée dans une colonne dont la température est maintenue entre 2 et 15°C.

13. Procédé selon l'une quelconque des revendications précédentes, **caractérisé en ce que** la solution aqueuse saline employée pour la mise en oeuvre de l'invention est une solution d'un chlorure d'un métal alcalin choisi parmi K+, Na+, Ca+, Mg+.

14. Procédé selon la revendication 12, **caractérisé en ce que** la solution aqueuse saline est une solution aqueuse de chlorure de sodium

15. Procédé selon la revendication 14, **caractérisé en ce que** la solution aqueuse saline est d'une concentration comprise entre 2 et 25% en poids de sel par poids de liquide.

16. Procédé selon la revendication 14, **caractérisé en ce que** la solution aqueuse saline a une force ionique comprise entre 1 et 2 M.

17. Procédé selon l'une quelconque des revendications précédentes, **caractérisé en ce que** le pH de la solution aqueuse saline d'élution est compris entre 6 et 7, avantageusement entre 6,5 et 7.

18. Procédé selon l'une quelconque des revendications précédentes, **caractérisé en ce que** le dessalage est fait par ultrafiltration et diafiltration.

19. Procédé selon la revendication 18, **caractérisé en ce que** les traitements d'ultrafiltration et de diafiltration sont effectués jusqu'à l'obtention d'un perméat ayant une conductivité inférieure à 15 mS.

20. Procédé selon l'une quelconque des revendications précédentes, **caractérisé en ce que** la stérilisation est faite par microfiltration.

21. Procédé selon l'une quelconque des revendications précédentes, **caractérisé en ce que** le produit dessalé et stérilisé est séché de façon à obtenir la fraction laitière issue du procédé de l'invention sous forme d'une poudre.

22. Fraction protéique laitière **caractérisée en ce qu'**elle est susceptible d'être obtenue par le procédé selon l'une quelconque des revendications 1 à 21.

23. Fraction protéique laitière, **caractérisée en ce qu'**elle répond aux caractéristiques suivantes :
- une teneur en protéine supérieure à 90%,
- une teneur en sels minéraux inférieure à 1 %,
- une teneur en matières grasses inférieure à 1%,
- une teneur en lactose inférieure à 1%,
- une teneur en humidité inférieure à 5%,
- une teneur en lactoferrine inférieure à 80%,
- un pH en solution à 2% compris entre 6 et 7,5,
- une pureté spectrophotométrique en UV-visible définie par un ratio DO⁴¹²/DO²⁸⁰<0,15,
- comporte au moins 1% de protéines ayant un point isoélectrique supérieur ou égal à 8,
les pourcentages étant donnés en poids par rapport au poids de matière sèche de la fraction laitière selon l'invention.

24. Fraction protéique laitière selon la revendication 23, **caractérisée en ce qu'**elle répond au moins à l'une des caractéristiques suivantes :
- une teneur en protéine supérieure à 95%,
- une teneur en sels minéraux inférieure à 0,5%,
- une teneur en matière grasse inférieure à 0,5%,
- une teneur en lactose inférieure à 0,5%,
- une teneur en humidité inférieure à 4%,
- une teneur en lactoferrine inférieure à 80%,
- un pH en solution à 2% compris entre 6 et 7,2,
- une pureté spectrophotométrique en UV-visible définie par un ratio DO⁴¹²/DO²⁸⁰<0,1.
- comporte au moins 1% de protéines ayant un point isoélectrique compris entre 8,2 et 8,7.

25. Fraction protéique laitière selon l'une quelconque des revendications 22 à 24, **caractérisée en ce qu'**elle est issue du lait de vache.

26. Fraction protéique laitière selon la revendication 25, **caractérisée en ce qu'**elle comporte au moins 40% de protéines ayant un point isoélectrique supérieur ou égal à 8.

27. Fraction protéique laitière selon la revendication 25 ou la revendication 26, **caractérisée en ce qu'**elle comporte une teneur en lactoferrine supérieure ou égale à 30% et une activité lactoperoxydase supérieure ou égale à 120 unités ABTS par mg d'isolat.

28. Fraction protéique laitière selon l'une quelconque des revendications 22 à 24, **caractérisée en ce qu'**elle est issue d'un lactosérum acide de caséine.

29. Association d'une fraction protéique laitière selon l'une quelconque des revendications 22 à 28 avec du calcium.

30. Association selon la revendication 29, **caractérisée en ce qu'**elle comporte en outre de la vitamine D.

31. Composition alimentaire, **caractérisée en ce qu'**elle comporte une fraction protéique laitière selon l'une quelconque des revendications 22 à 30.

32. Kit alimentaires comprenant une poudre de fraction protéique laitière selon l'une quelconque des revendications 22 à 30.

33. Utilisation d'une fraction protéique laitière selon l'une quelconque des revendications 22 à 30, pour la préparation d'un lait alimentaire

34. Utilisation d'une fraction protéique laitière selon l'une quelconque des revendications 22 à 30, pour la préparation d'un aliment destiné à la prévention d'une pathologies sélectionnée parmi : retard de croissance, ostéoporose, fragilité osseuse, fractures osseuses, rhumatismes, arthrose, maladies périodontales, déficience de la barrière intestinale.

35. Utilisation d'une fraction protéique laitière selon l'une quelconque des revendications 22 à 30 pour la préparation d'un aliment destiné à favoriser la croissance des ostéoblastes et/ou des cellules intestinales et/ou à inhiber la croissance des pré-ostéoclastes.

36. Composition pharmaceutique **caractérisée en ce qu'**elle comprend au moins une fraction protéique laitière selon l'une quelconque des revendications 22 à 30 et un support pharmaceutiquement acceptable.

37. Utilisation d'une fraction protéique laitière selon l'une quelconque des revendications 22 à 30, pour la préparation d'un médicament destiné à la prévention et/ou au traitement d'une pathologies sélectionnée parmi : retard de croissance, ostéoporose, fragilité osseuse, fractures osseuses, rhumatismes, arthrose, maladies périodontales, déficience de la barrière intestinale.

38. Utilisation d'une fraction protéique laitière selon l'une quelconque des revendications 22 à 30, pour la préparation d'un médicament destiné à améliorer l'absorption du calcium dans l'organisme.

39. Utilisation d'une fraction protéique laitière selon l'une quelconque des revendications 22 à 30 pour la préparation d'un médicament destiné à favoriser la croissance des ostéoblastes et/ou des cellules intestinales et/ou à inhiber la croissance des pré-ostéoclastes.

40. Produit d'hygiène **caractérisé en ce qu'**il comprend au moins une fraction protéique laitière selon l'une quelconque des revendications 22 à 30.

## Claims

1. A method for isolating milk proteins from milk or a lactoserum, comprising the following stages:
a) the milk or lactoserum is sterilised and defatted;
b) the milk fraction resulting from stage a) is fed to a cation exchanger resin conditioned in an elution column;
c) the fraction retained on the resin is eluted by a saline aqueous solution;
d) the eluate resulting from stage c) is desalinated and sterilised.
this process being **characterised in that**:
α) the cation exchange resin is a resin grafted by strong acid functions;
parameter BV denoting the ratio of the volume of raw material to the volume of wet resin in the column,
parameter SV denoting the ratio of the supply flow rate to the column to the volume of wet resin in the column,
parameter LV denoting the ratio of the supply flow rate to the column to the cross-section of the column,
β) during stage b), the fixing parameters have the following values:
- BV_{f} is between 50 and 400;
- SV_{f} is between 2 and 40 h⁻¹;
- LV_{f} is greater than or equal to 1 m/h and less than or equal to 5 m/h.
γ) during stage c), the elution parameters have the following values:
- BV₃ is between 1.5 and 7;
- LVₑ is less than 1 m/h.

2. The method according to Claim 1, **characterised in that** the initial product is cow's milk.

3. The method according to Claim 1, **characterised in that** the initial product is an acid casein lactoserum.

4. The method according to any one of the preceding claims, **characterised in that** the cation exchange resin is a resin grafted by acid functions of pKa ≤ 2, having an ion exchange capacity of between 200 and 1000 µE/ml.

5. The method according to Claim 4, **characterised in that** the resin is grafted by sulphonic acid or sulphonate salt functions.

6. The method according to Claim 5, **characterised in that** the resin is grafted by sulphonic propyl or sulphonate propyl functions.

7. The method according to any one the preceding claims, **characterised in that** the granulometry of the resin is between 100 µm and 900 µm, preferably between 200 and 750 µm, and even more preferably between 250 and 600 µm.

8. The method according to any one of the preceding claims, **characterised in that** during stage b) of fixing of the raw material, one or more of the following conditions are met:
- BV_{f} is between 80 and 150;
- SV_{f} is between 5 and 40 h-¹;
- LV_{f} is between 3 and 4.3 m/h.

9. The method according to any one of the preceding claims, **characterised in that** the following conditions are verified
during stage b):
- BV_{f} is between 80 and 120;
- SV_{f} is between 8 and 15 h⁻¹;
- LV_{f} is between 3 and 4.8 m/h,
during stage c):
- BVₑ is between 3 and 7
- LVₑ is less than 1 m/h.

10. The method according to any one of the preceding claims, **characterised in that** during stage b) the resin is conditioned in a column whose temperature is maintained between 2 and 15°C.

11. The method according to any one of the preceding claims, **characterised in that** during stage c) of elution of the fixed proteins, at least one of the following conditions is met:
- BVₑ is between 3 and 5;
- LVₑ is inferior 0.5 m/h,

12. The method according to any one of the preceding claims, **characterised in that** during stage c) the resin is conditioned in a column whose temperature is maintained between 2 and 15°C.

13. The method according to any one of the preceding claims, **characterised in that** the saline aqueous solution used for implementing the invention is a solution of a chloride of an alkaline metal selected from K+, Na+, Ca+, Mg+.

14. The method according to Claim 12, **characterised in that** the saline aqueous solution is an aqueous solution of sodium chloride.

15. The method according to Claim 14, **characterised in that** the saline aqueous solution has a concentration of between 2 and 25% by weight of salt by weight of liquid.

16. The method according to Claim 14, **characterised in that** the saline aqueous solution has an ionic force of between 1 and 2 M.

17. The method according to any one of the preceding claims, **characterised in that** the pH of the saline aqueous solution of elution is between 6 and 7, advantageously between 6.5 and 7.

18. The method according to any one of the preceding claims, **characterised in that** the desalination is carried out by ultrafiltration and diafiltration.

19. The method according to Claim 18, **characterised in that** the ultrafiltration and diafiltration treatments are carried out until a permeate is obtained that has a conductivity lower than 15 mS.

20. The method according to any one of the preceding claims, **characterised in that** the sterilisation is carried out by microfiltration.

21. The method according to any one of the preceding claims, **characterised in that** the desalinated and sterilised product is dried in such a manner that the milk fraction resulting from the method of the invention is obtained in the form of a powder.

22. A proteic milk fraction **characterised in that** it is capable of being obtained by the method according to any one of Claims 1 to 21.

23. A proteic milk fraction **characterised in that** it has the following characteristics:
- a protein content higher than 90%,
- a content of mineral salts lower than 1%,
- a content of fats lower than 1%,
- a lactose content lower than 1%,
- a moisture content lower than 5%,
- a lactoferrin content lower than 80%,
- a pH in a 2% solution of between 6 and 7.5,
- a spectrophotometric purity in the visible UV defined by a ratio DO⁴¹²/DO²⁸⁰<0.15,
- contains at least 1% of proteins having an isoelectric point greater than or equal to 8,
- the percentages being given by weight in relation to the weight of dry matter of the milk fraction according to the invention.

24. Proteic milk fraction according to Claim 23, **characterised in that** it has at least one of the following characteristics:
- a protein content higher than 95%,
- a content of mineral salts lower than 0.5%,
- a content of fats lower than 0.5%,
- a lactose content lower than 0.5%,
- a moisture content lower than 4%,
- a lactoferrin content lower than 80%,
- a pH in a 2% solution of between 6 and 7.2,
- a spectrophotometric purity in the visible UV defined by a ratio DO⁴¹²/DO²⁸⁰<0.1,
- contains at least 1% of proteins having an isoelectric point between 8.2 and 8.7.

25. The proteic milk fraction according to any one of Claims 22 to 24, **characterised in that** it derives from cow's milk.

26. The proteic milk fraction according to Claim 25, **characterised in that** it contains at least 40% of proteins having an isoelectric point greater than or equal to 8.

27. The proteic milk fraction according to Claim 25 or Claim 26, **characterised in that** it has a lactoferrin content higher than or equal to 30% and a lactoperoxydase activity higher than or equal to 120 units ABTS per mg of isolate.

28. The proteic milk fraction according to any one of Claims 22 to 24, **characterised in that** it derives from a casein acid lactoserum.

29. An association of a proteic milk fraction according to any one of Claims 22 to 28 with calcium.

30. The association according to Claim 29, **characterised in that** it also contains vitamin D.

31. A food composition, **characterised in that** it contains a proteic milk fraction according to any of Claims 22 to 30.

32. A food kit comprising a powder of proteic milk fraction according to any one of Claims 22 to 30.

33. The use of a proteic milk fraction according to any one of Claims 22 to 30 for the preparation of food grade milk.

34. The use of a proteic milk fraction according to any one of Claims 22 to 30 for the preparation of a food intended to prevent one of the pathologies selected from: growth retardation, osteoporosis, brittle bone disease, bone fractures, rheumatism, arthritis, periodontal diseases, intestinal barrier deficiency.

35. The use of a proteic milk fraction according to any one of Claims 22 to 30 for the preparation of a food intended to promote the growth of osteoblasts and/or intestinal cells and/or to inhibit the growth of pre-osteoclasts.

36. A pharmaceutical composition **characterised in that** it comprises at least one proteic milk fraction according to any one of Claims 22 to 30 and a pharmaceutically acceptable medium.

37. The use of a proteic milk fraction according to any one of Claims 22 to 30 for the preparation of a medicinal product intended to prevent and/or treat one of the pathologies selected from: growth retardation, osteoporosis, brittle bone disease, bone fractures, rheumatism, arthritis, periodontal diseases, intestinal barrier deficiency.

38. The use of a proteic milk fraction according to any one of Claims 22 to 30 for the preparation of a medicinal product intended to improve the absorption of calcium in the organism.

39. The use of a proteic milk fraction according to any one of Claims 22 to 30 for the preparation of a medicinal product intended to promote the growth of osteoblasts and/or the intestinal cells and/or to inhibit the growth of pre-osteoclasts.

40. A hygiene product **characterised in that** it contains at least a proteic milk fraction according to any one of Claims 22 to 30.

## Patentansprüche

1. Verfahren zur Isolierung von Milchproteinen aus Milch oder einer Molke, umfassend die folgenden Stufen:
a) die Milch oder die Molke wird sterilisiert und entfettet;
b) die Milchfraktion aus Stufe a) wird über ein konditioniertes Kationenaustauschharz in einer Elutionssäule geführt;
c) die auf dem Harz zurückgehaltene Fraktion wird durch eine wässrige Salzlösung eluiert;
d) das aus Stufe c) resultierende Eluat wird entsalzt und sterilisiert;
wobei dieses Verfahren **dadurch gekennzeichnet ist, dass**
α) das Kationenaustauschharz ein Harz ist, das mit starken sauren Funktionen gepfropft ist;
der Parameter BV das Verhältnis des Volumens an Ausgangsmaterial zu dem Volumen des feuchten Harzes in der Säule bezeichnet;
der Parameter SV das Verhältnis der Durchflussmenge der Säule zum Volumen des feuchten Harzes in der Säule bezeichnet;
der Parameter LV das Verhältnis der Durchflussmenge zu dem Querschnitt der Säule bezeichnet;
β) im Verlauf der Stufe b) die Fixierungsparameter die folgenden Werte haben:
- BV_{f} liegt zwischen 50 und 400;
- SV_{f} liegt zwischen 2 und 40 h⁻¹;
- LV_{f} ist höher oder gleich 1 m/h und unter oder gleich 5 m/h;
γ) im Verlauf der Stufe c) die Elutionsparameter die folgende Werte haben:
- BVₑ liegt zwischen 1,5 und 7;
- LVₑ liegt unter 1 m/h.

2. Verfahren nach Anspruch 1, **dadurch gekennzeichnet, dass** das Ausgangsprodukt Kuhmilch ist.

3. Verfahren nach Anspruch 1, **dadurch gekennzeichnet, dass** das Ausgangsprodukt eine Casein-Sauermolke (bzw. Caseinmolke) ist.

4. Verfahren nach einem der vorangehenden Ansprüche, **dadurch gekennzeichnet, dass** das Kationenaustauschharz ein Harz ist, das mit sauren Funktionen mit pKs ≤ 2 gepfropft ist, das eine Ionenaustauschkapazität zwischen 200 und 1000 µe/ml hat.

5. Verfahren nach Anspruch 4, **dadurch gekennzeichnet, dass** das Harz mit Sulfonsäure- oder Sulfonatsalz-Funktionen gepfropft ist.

6. Verfahren nach Anspruch 5, **dadurch gekennzeichnet, dass** das Harz mit Propylsulfon- oder Propylsulfonat-Funktionen gepfropft ist.

7. Verfahren nach einem der vorangehenden Ansprüche, **dadurch gekennzeichnet, dass** die Granulometrie des Harzes zwischen 100 µm und 900 µm, vorzugsweise zwischen 200 und 750 µm, noch bevorzugter zwischen 250 und 600 µm, liegt.

8. Verfahren nach einem der vorangehenden Ansprüche, **dadurch gekennzeichnet, dass** im Verlauf der Stufe b) der Fixierung des Ausgangsmaterials eine oder mehrere der folgenden Bedingungen erfüllt ist/sind:
- BV_{f} liegt zwischen 80 und 150;
- SV_{f} liegt zwischen 5 und 40 h⁻¹;
- LV_{f} liegt zwischen 3 und 4,3 m/h.

9. Verfahren nach einem der vorangehenden Ansprüche, **dadurch gekennzeichnet, dass** die folgenden Bedingungen eingehalten werden:
im Verlauf der Stufe b):
- BV_{f} liegt zwischen 80 und 120;
- SV_{f} liegt zwischen 8 und 15 h⁻¹;
- LV_{f} liegt zwischen 3 und 4,8 m/h;
im Verlauf der Stufe c):
- BVₑ liegt zwischen 3 und 7;
- LVₑ ist weniger als 1 m/h.

10. Verfahren nach einem der vorangehenden Ansprüche, **dadurch gekennzeichnet, dass** im Verlauf der Stufe b) das Harz in einer Säule konditioniert wird, deren Temperatur zwischen 2 und 15°C gehalten wird.

11. Verfahren nach einem der vorangehenden Ansprüche, **dadurch gekennzeichnet, dass** im Verlauf der Stufe c) der Elution der fixierten Proteine wenigstens eine der folgenden Bedingungen erfüllt ist:
- BVₑ liegt zwischen 3 und 5;
- LVₑ ist kleiner als 0,5 m/h.

12. Verfahren nach einem der vorangehenden Ansprüche, **dadurch gekennzeichnet, dass** im Verlauf der Stufe c) das Harz in einer Säule konditioniert wird, deren Temperatur zwischen 2 und 15°C gehalten wird.

13. Verfahren nach einem der vorangehenden Ansprüche, **dadurch gekennzeichnet, dass** die wässrige Salzlösung, die zur Durchführung der Erfindung verwendet wird, eine Lösung eines Chlorids eines Alkalimetalls ist, das unter K⁺, Na⁺, Ca⁺, Mg⁺ ausgewählt wird.

14. Verfahren nach Anspruch 12, **dadurch gekennzeichnet, dass** die wässrige Salzlösung eine wässrige Natriumchloridlösung ist.

15. Verfahren nach Anspruch 14, **dadurch gekennzeichnet, dass** die wässrige Salzlösung eine Konzentration zwischen 2 und 25 Gew.-% Salz pro Gewicht Flüssigkeit hat.

16. Verfahren nach Anspruch 14, **dadurch gekennzeichnet, dass** die wässrige Salzlösung eine Ionenstärke zwischen 1 und 2 M hat.

17. Verfahren nach einem der vorangehenden Ansprüche, **dadurch gekennzeichnet , dass** der pH der wässrigen Salzlösung zur Elution zwischen 6 und 7, vorzugsweise zwischen 6,5 und 7 liegt.

18. Verfahren nach einem der vorangehenden Ansprüche, **dadurch gekennzeichnet, dass** die Entsalzung durch Ultrafiltration und Diafiltration erfolgt.

19. Verfahren nach Anspruch 18, **dadurch gekennzeichnet, dass** die Behandlungen der Ultrafiltration und der Diafiltration bis zum Erhalt eines Permeats mit einer Leitfähigkeit von unter 15 mS durchgeführt werden.

20. Verfahren nach einem der vorangehenden Ansprüche, **dadurch gekennzeichnet, dass** die Sterilisation durch Mikrofiltration durchgeführt wird.

21. Verfahren nach einem der vorangehenden Ansprüche, **dadurch gekennzeichnet, dass** das entsalzte und sterilisierte Produkt derart getrocknet wird, dass die Milchfraktion, die aus dem Verfahren der Erfindung stammt, in Form eines Pulvers erhalten wird.

22. Milchproteinfraktion, **dadurch gekennzeichnet, dass** sie durch das Verfahren nach einem der Ansprüche 1 bis 21 erhalten werden kann.

23. Milchproteinfraktion, **dadurch gekennzeichnet, dass** sie den folgenden Charakteristika entspricht:
- ein Proteingehalt über 90%,
- ein Gehalt an Mineralsalzen unter 1%,
- ein Fettgehalt unter 1%,
- ein Gehalt an Lactose unter 1%,
- ein Feuchtigkeitsgehalt unter 5%,
- ein Gehalt an Lactoferrin unter 80%,
- ein pH in einer Lösung mit 2% zwischen 6 und 7,5,
- eine spektralphotometrische Reinheit im UV-sichtbaren Bereich, definiert durch das Verhältnis OD⁴¹² /OD²⁸⁰, < 0, 15,
- enthält wenigstens 1% Proteine mit einem isoelektrischen Punkt über oder gleich 8,
wobei die Prozentangaben als Gewicht bezogen auf das Gewicht der Trockensubstanz der Milchfraktion gemäß der Erfindung angegeben sind.

24. Milchproteinfraktion nach Anspruch 23, **dadurch ge-kennzeichnet, dass** sie wenigstens einem der folgenden Charakteristika entspricht:
- ein Proteingehalt über 95%,
- ein Gehalt an Mineralsalzen unter 0,5%,
- ein Fettgehalt unter 0,5%,
- ein Lactosegehalt unter 0,5%,
- ein Feuchtigkeitsgehalt unter 4%,
- ein Gehalt an Lactoferrin unter 80%,
- ein pH in Lösung mit 2% zwischen 6 und 7,2,
- eine spektralphotometrische Reinheit im UV-sichtbaren Bereich, definiert durch das Verhältnis OD⁴¹²/OD²⁸⁰, < 0,1,
- umfasst wenigstens 1% Proteine mit einem isoelektrischen Punkt zwischen 8,2 und 8,7.

25. Milchproteinfraktion nach einem der Ansprüche 22 bis 24, **dadurch gekennzeichnet, dass** sie aus Kuhmilch stammt.

26. Milchproteinfraktion nach Anspruch 25, **dadurch gekennzeichnet, dass** sie wenigstens 40% Proteine mit einem isoelektrischen Punkt über oder gleich 8 umfasst.

27. Milchproteinfraktion nach Anspruch 25 oder Anspruch 26, **dadurch gekennzeichnet, dass** sie einen Gehalt an Lactoferrin über oder gleich 30% und eine Lactoperoxidaseaktivität über oder gleich 120 ABTS-Einheiten pro mg Isolat umfasst.

28. Milchproteinfraktion nach einem der Ansprüche 22 bis 24, **dadurch gekennzeichnet, dass** sie von einer Casein-Sauermolke (bzw. Caseinmolke) abgeleitet ist.

29. Kombination einer Milchproteinfraktion nach einem der Ansprüche 22 bis 28 mit Calcium.

30. Kombination nach Anspruch 29, **dadurch gekennzeichnet, dass** sie außerdem Vitamin D umfasst.

31. Alimentäre Zusammensetzung, **dadurch gekennzeichnet, dass** sie eine Milchproteinfraktion nach einem der Ansprüche 22 bis 30 umfasst.

32. Alimentärer Kit, der ein Pulver der Milchproteinfraktion nach einem der Ansprüche 22 bis 30 umfasst.

33. Verwendung einer Milchproteinfraktion nach einem der Ansprüche 22 bis 30 für die Herstellung einer alimentären Milch.

34. Verwendung einer Milchproteinfraktion nach einem der Ansprüche 22 bis 30 für die Herstellung eines Nahrungsmittels, das zur Prävention einer Pathologie bestimmt ist, die ausgewählt ist unter: Wachstumsretardierung, Osteoporose, Knochenbrüchigkeit, Knochenbrüchen, Rheumatismus, Arthrose, periodontalen Krankheiten, Defizienz der Intestinalschranke.

35. Verwendung einer Milchproteinfraktion nach einem der Ansprüche 22 bis 30 für die Herstellung eines Nahrungsmittels, das dazu bestimmt ist, das Wachstum der Osteoblasten und/oder der Intestinalzellen zu begünstigen und/oder das Wachstum der Präosteoklasten zu inhibieren.

36. Pharmazeutische Zusammensetzung, **dadurch gekennzeichnet, dass** sie wenigstens eine Milchproteinfraktion nach einem der Ansprüche 22 bis 30 und einen pharmazeutisch annehmbaren Träger umfasst.

37. Verwendung einer Milchproteinfraktion nach einem der Ansprüche 22 bis 30 für die Herstellung eines Medikaments, das zur Prävention und/oder der Behandlung einer Pathologie bestimmt ist, die ausgewählt ist unter: Wachstumsretardierung, Osteoporose, Knochenbrüchigkeit, Knochenbrüchen, Rheumatismus, Arthrose, periodontalen Krankheiten, Defizienz der Intestinalbarriere.

38. Verwendung einer Milchproteinfraktion nach einem der Ansprüche 22 bis 30 für die Herstellung eines Medikaments, das dazu bestimmt ist, die Absorption von Calcium im Organismus zu verbessern.

39. Verwendung einer Milchproteinfraktion nach einem der Ansprüche 22 bis 30 für die Herstellung eines Medikaments, das dazu bestimmt ist, das Wachstum der Osteoblasten und/oder der Intestinalzellen zu verbessern und/oder das Wachstum der Präosteoklasten zu inhibieren.

40. Hygieneprodukt, **dadurch gekennzeichnet, dass** es wenigstens eine Milchproteinfraktion nach einem der Ansprüche 22 bis 30 umfasst.
